# EUROPÄISCHE PATENTSCHRIFT

(11) **EP 0 894 019 B1**
(45) Veröffentlichungstag und Bekanntmachung des Hinweises auf die Patenterteilung: **05.11.2003**
(21) Anmeldenummer: 97921592.8
(22) Anmeldetag: 27.03.1997
(51) Int. Cl.: A61N 7/02, A61B 8/12

(54) **AKUSTISCHES THERAPIEGERÄT AUFWEISEND EINE QUELLE THERAPEUTISCHER AKUSTISCHER WELLEN UND EINE ULTRASCHALL-ORTUNGSEINRICHTUNG**
ACOUSTIC THERAPY DEVICE WITH A SOURCE OF THERAPEUTIC ACOUSTIC WAVES AND AN ULTRASOUND LOCATION SYSTEM
APPAREIL THERAPEUTIQUE ACOUSTIQUE PRESENTANT UNE SOURCE D'ONDES ACOUSTIQUES THERAPEUTIQUES ET UN DISPOSITIF DE LOCALISATION A ULTRASONS

(30) Priorität: 02.04.1996 DE 19613243
(43) Veröffentlichungstag der Anmeldung: 03.02.1999
(73) Patentinhaber: SIEMENS AKTIENGESELLSCHAFT, 80333 München (DE)
(72) Erfinder: SCHÄTZLE, Ulrich, D-91341 Röttenbach (DE); SCHMIDT, Erhard, D-91054 Erlangen (DE); THUM, Bernhard, D-89359 Koetz (DE)
(86) Internationale Anmeldenummer: DE9700632
(87) Internationale Veröffentlichungsnummer: WO97036650

(56) Entgegenhaltungen:
- EP-A- 0 668 052
- WO-A-93/16641
- WO-A-95/02994
- WO-A-95/19143

## Beschreibung

Die Erfindung betrifft ein akustisches Therapiegerät, aufweisend eine Quelle therapeutischer akustischer Wellen und eine Ultraschall-Ortungseinrichtung mit einem Ultraschall-Transducer, wobei die Quelle und der Ultraschall-Transducer derart zu einem länglichen, zur Einführung in das Körperinnere eines Patienten vorgesehenen Applikator mit einer Längsachse zusammengefaßt sind, daß die Quelle und der Ultraschall-Transducer in Richtung der Längsachse aufeinanderfolgend angeordnet sind, wobei mittels der Ultraschall-Ortungseinrichtung Ultraschall-Bilder bezüglich einer kreissektorförmigen, die Längsachse des Applikators enthaltenden Schnittebene erzeugbar sind, deren Mittelachse die Winkelhalbierende der Schnittebene ist.

Um denjenigen Bereich, der mittels der therapeutischen akustischen Wellen behandelt werden soll, mittels der Ultraschall-Ortungseinrichtung gut abbilden zu können ist bei herkömmlichen Therapiegeräten der eingangs genannten Art die Quelle therapeutischer akustischer Wellen in zwei gleich lange Abschnitte unterteilt, zwischen denen sich der Sektor-Scanner befindet. Dabei enthält der Sektor-Scanner meist ein kleines Linear Array, das nach Art eines Phased Array betrieben wird. Ein ein Linear Array enthaltender Sektor-Scanner wird einerseits deshalb verwendet, weil es dann wegen des geringen Bauraumbedarfes eines solchen Sektor-Scanners möglich ist, die beiden Abschnitte der Quelle therapeutischer akustischer Wellen dicht beieinander anzuordnen, was aus akustischen Gründen günstig ist. Andererseits wirkt sich der geringe Bauraumbedarf eines solchen Sektor-Scanners günstig auf die Abmessungen des Applikators aus, so daß ein Einsatz des Applikators auch unter beengten Platzverhältnissen, z.B. endoluminale Applikation (Einsatz in Körperhöhlen), möglich ist.

Aus der US 5 474 071 ist ein Therapiegerät der eingangs genannten Art bekannt, bei dem die Quelle therapeutischer akustischer Wellen und der Ultraschall-Transducer in Richtung der Längsachse aufeinanderfolgend angeordnet sind, wobei mittels der Ultraschall-Ortungseinrichtung Ultraschall-Bilder bezüglich einer Schnittebene erzeugbar sind, die die Längsachse des Applikators enthält, und wobei die Mittelachse der Schnittebene mit der Längsachse des Applikators in einer solchen Weise einen Winkel einschließt, der kleiner als 90° ist, daß die Mittelachse zu der Quelle hin geneigt ist. Auf diese Weise ist es möglich, in dem mittels der Ultraschall-Ortungseinrichtung erzeugten Ultraschallbild stets den gesamten mittels der therapeutischen akustischen Wellen zu behandelnden Bereich abzubilden, ohne daß eine Unterteilung der Quelle therapeutischer akustischer Wellen in zwei Abschnitte erforderlich ist. Allerdings steht für den Ultraschall-Transducer nur sehr wenig Bauraum zur Verfügung.

Der Erfindung liegt die Aufgabe zugrunde, ein Therapiegerät der eingangs genannten Art so auszubilden, daß es ohne Unterteilung der Quelle therapeutischer akustischer Wellen in zwei Abschnitte möglich ist, den mittels der therapeutischen akustischen Wellen zu behandelnden Bereich mittels der Ultraschall-Ortungseinrichtung gut abzubilden, und dennoch ausreichend Bauraum für den Ultraschall-Transducer zur Verfügung steht.

Nach der Erfindung wird diese Aufgabe durch das Gerät gemäß Anspruch 1 gelöst. Es wird also deutlich, daß der Sektor-Scanner am distalen Ende des Applikators angeordnet ist, mit der Folge, daß ausreichend Bauraum für den Sektor-Scanner zur Verfügung steht. Dennoch ist es infolge der Neigung der Mittelachse der Schnittebene möglich, in dem mittels der Ultraschall-Ortungseinrichtung erzeugten Ultraschallbild stets den gesamten mittels der therapeutischen akustischen Wellen zu behandelnden Bereich abzubilden.

Außerdem ist es möglich, während der Einführung des Applikators in einen Patienten mittels der Ultraschall-Ortungseinrichtung den während des Einführvorgangs im Bereich des distalen Endes des Applikators, insbesondere vor dem distalen Ende, befindlichen Körperbereich des Patienten darzustellen, und zwar um Informationen zu erhalten, welche es gestatten zu erkennen, ob eine problemlose Einführung möglich ist oder ob beispielsweise die Einführrichtung korrigiert werden muß, da gemäß der Erfindung vorgesehen ist, daß die Mittelachse der Schnittebene derart verstellbar ist, daß sie mit dem im Bereich der Quelle befindlichen Abschnitt der Längsachse des Applikators einen Winkel einschließt, der größer als 90° ist.

Infolge des erfindungsgemäßen Aufbaues ist es in vorteilhafter Weise selbst bei Verwendung eines mechanischen Sektor-Scanners mit geringem technischen Aufwand möglich, dem Applikator solche Abmessungen zu geben, daß er auf natürlichem Wege in das Körperinnere eines menschlichen Patienten einführbar ist.

Die Anordnung des Ultraschall-Transducers im Sinne der Einführrichtung vor der Quelle, d.h. am distalen Ende des Applikators, bietet den Vorteil, daß die Ultraschall-Ortungseinrichtung auch bei endoluminaler Applikation unter allen Umständen ein gutes Bild liefert.

Ein Ausführungsbeispiel der Erfindung ist in den beigefügten Zeichnungen am Beispiel eines diagnostischen Ultraschall-Applikators dargestellt. Es zeigen:
- Fig. 1: einen teilweisen Längsschnitt durch ein einen mechanischen Sektor-Scanner enthaltenden erfindungsgemäßes Therapiegerät und in blockschaltbildartiger, schematischer Darstellung die zum Betrieb des Therapiegerätes erforderlichen Einrichtungen sowie eine mit dem Ultraschall-Applikator als Ultraschall-Ortungseinrichtung zusammenwirkendes Ultraschallgerät,
- Fig. 2: und 3 Schnitte gemäß den Linien II-II und III-III in Fig. 1,
- Fig. 4: in perspektivischer Darstellung den Rotor des in dem Ultraschall-Applikator gemäß den Fig. 1 bis 3 enthaltenen erfindungsgemäßen Sektor-Scanners,
- Fig. 5: in perspektivischer Darstellung die in dem Therapiegerät gemäß den Fig. 1 bis 3 enthaltene Quelle therapeutischer Ultraschallwellen, und
- Fig. 6: in perspektivischer Darstellung den UltraschallApplikator des Therapiegerätes gemäß den Fig. 1 bis 3.

Das erfindungsgemäße akustisches Therapiegerät weist eine Quelle 1 therapeutischer akustischer Wellen und eine Ultraschall-Ortungseinrichtung mit einem mechanischen Sektor-Scanner 2 auf.

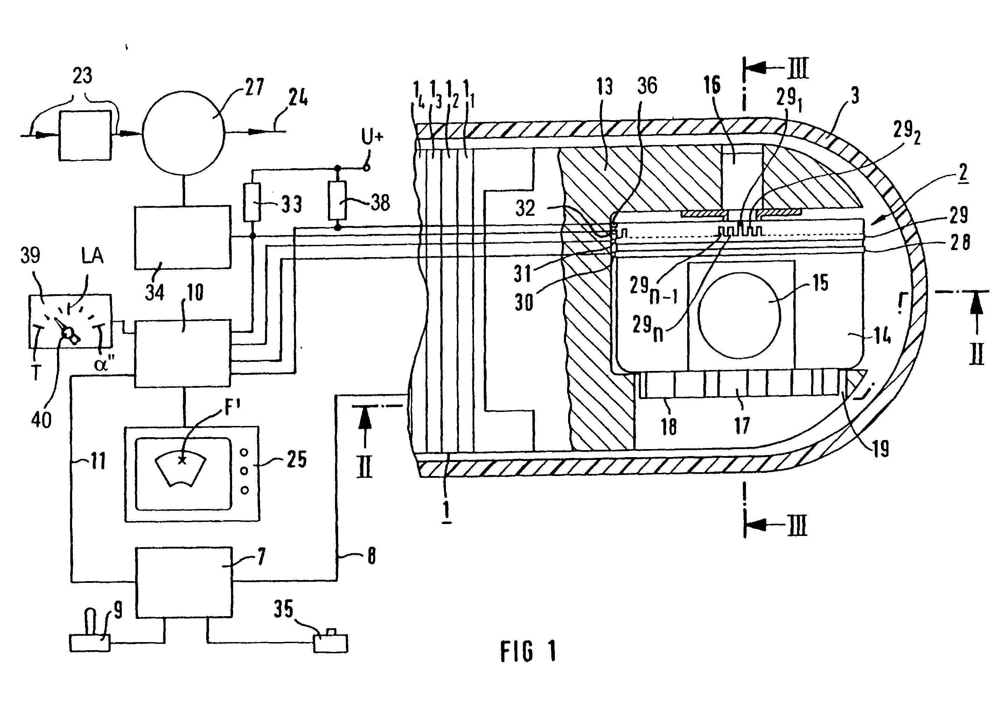

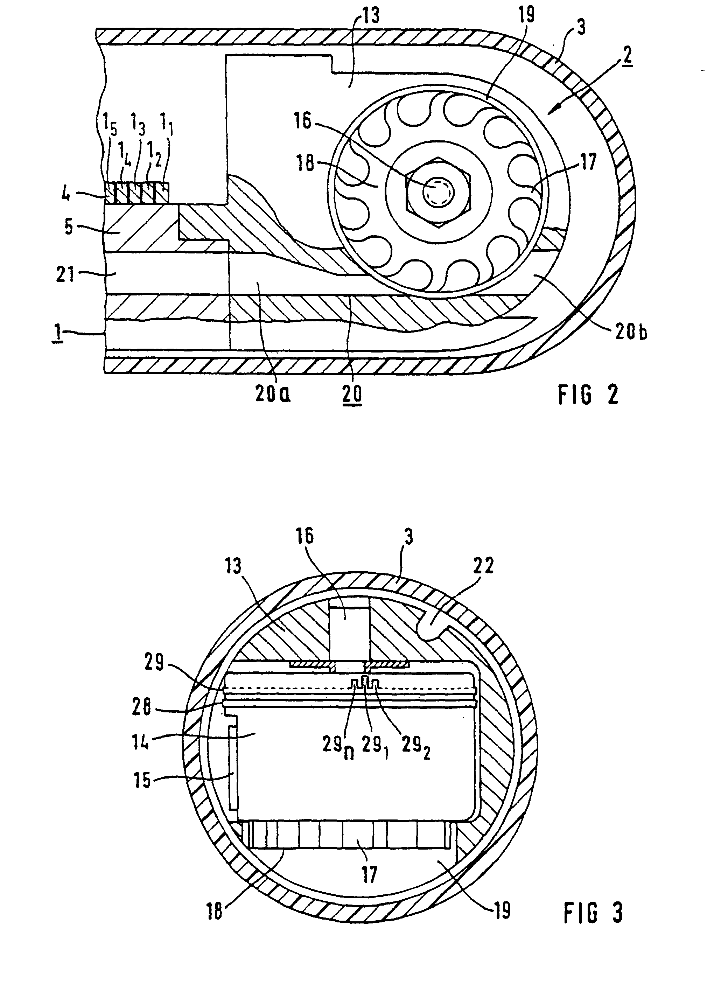

Wie aus den Fig. 1 und 2 ersichtlich ist, sind die Quelle 1 therapeutischer akustischer Wellen und der Sektor-Scanner 2 zu einem länglichen Applikator zusammengefaßt. Dieser weist ein rohrförmiges, an seinem distalen, also zur Einführung in eine vorzugsweise natürliche Körperöffnung vorgesehenen Ende kuppelförmig ausgebildetes Gehäuse 3 auf, das aus einem für Ultraschallwellen gut durchlässigen Material, beispielsweise aus Kunststoff, z.B. Polymethylpenten (TPX®) gebildet ist.

Der Innenraum des Applikators bzw. des Gehäuses 3 ist in nicht dargestellter Weise mit einer als Ausbreitungsmedium für Ultraschall geeigneten Flüssigkeit, beispielsweise leichtem Mineralöl, gefüllt.

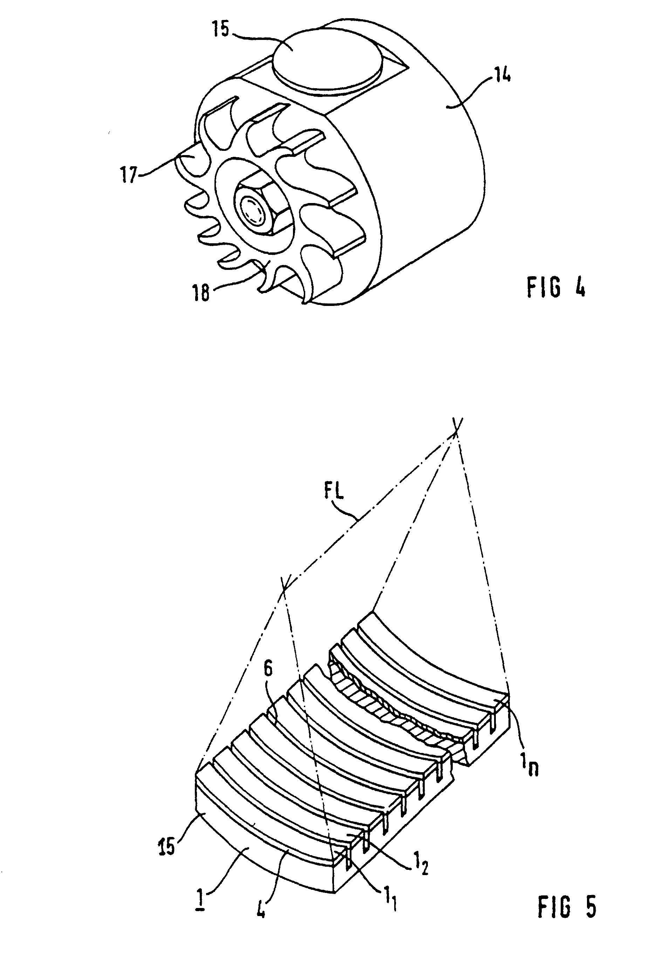

Bei der Quelle 1 handelt es sich um eine langgestreckte piezoelektrische Quelle, die gemäß Fig. 5 als sogenanntes Linear Array aufgebaut ist und während der Behandlung eines Patienten mittels des Therapiegerätes Ultraschallwellen abgibt.

Demnach ist die Quelle 1 in eine Vielzahl von Ultraschall-Wandlerelementen 1₁, 1₂ usw. bis 1ₙ unterteilt. Die Unterteilung ist derart ausgeführt, daß es möglich ist, jedes der Ultraschall-Wandlerelemente 1₁ bis 1ₙ durch Zufuhr eines geeigneten elektrischen Signals einzeln zur Erzeugung von Ultraschallwellen anzutreiben. Der Übersichtlichkeit halber ist die Quelle 1 in der nicht maßstabsgetreuen Fig. 5 verkürzt dargestellt, so daß nur einige wenige, nämlich ca. 10 Ultraschall-Wandlerelemente dargestellt sind. In der Praxis ist die Quelle 1 in beispielsweise 128, 192 oder 256 Ultraschallwandlerelemente unterteilt und weist eine Länge von einigen Zentimetern auf.

Die Quelle 1 ist in an sich bekannter Weise derart aufgebaut, daß das eigentliche piezoelektrische Material 4 in Form einer Schicht konstanter Dicke auf einen Tragkörper 5 geeigneter akustischer Impedanz und ebenfalls konstanter Dicke aufgebracht ist. Dabei erfolgt die Verbindung der Schicht 4 piezoelektrischen Materials mit dem Tragkörper 5 in nicht dargestellter Weise durch eine metallische Schicht, deren Dicke klein im Verhältnis zu der der Schicht 4 ist. Die von dem Tragkörper 5 abgewandte Fläche der piezoelektrischen Schicht 4 ist ebenfalls mit einer dünnen, nicht dargestellten metallischen Schicht versehen.

Die genannten metallischen Schichten, dienen als Elektroden zur elektrischen Kontaktierung der Ultraschall-Wandlerelemente 1₁ bis 1ₙ.

Um Ultraschall-Wandlerelemente 1₁ bis 1ₙ zu erhalten, die unabhängig von einander ansteuerbar sind, ist die mit dem Tragkörper 5 verbundene piezoelektrische Schicht 4 durch quer zur Längsachse der Quelle 1 verlaufende schmale Einschnitte, einer davon ist in Fig. 5 mit 6 bezeichnet, in die einzelnen Ultraschall-Wandlerelemente 1₁ bis 1ₙ unterteilt.

Um die Ultraschall-Wandlerelemente 1₁ bis 1ₙ mechanisch von einander zu entkoppeln, weisen die Einschnitte 6 eine Tiefe auf, die deutlich größer als die Dicke der piezoelektrischen Schicht 4 ist.

Bei geeigneter Ansteuerung der einzelnen Ultraschall-Wandlerelemente 1₁ bis 1ₙ ist es möglich, die von der Quelle 1 abgestrahlten Ultraschallwellen auf eine Fokuszone zu fokussieren und die Fokuszone der Ultraschallwellen zu verlagern. Bekanntermaßen ist auf diesem Wege eine Fokussierung bzw. die Ausführung einer Abtastbewegung nur in Richtung der Längsachse des Linear Array sowie in Richtung der Schallausbreitung orthogonal zur Oberfläche des Ultraschall-Wandlers 15 möglich. Um eine Fokussierung auch quer dazu zu erreichen, ist der Ul-traschall-Wandler 15 in der in Fig. 2 angedeuteten, an sich bekannten Weise um eine parallel zu seiner Längsachse verlaufende Achse zylindrisch gekrümmt, so daß sich bei gleichzeitiger Ansteuerung aller Ultraschall-Wandlerelemente 1₁ bis 1ₙ eine Fokussierung der Ultraschallwellen auf einen in Fig. 5 mit FL bezeichneten Linienfokus ergibt, der parallel zu der Längsachse des Linear Array verläuft.

Werden dagegen die Ultraschall-Wandlerelemente 1₁ bis 1ₙ mit zu einander in geeigneter Weise phasenversetzten Signalen angesteuert, so erfolgt eine Fokussierung auf eine annähernd elipsoidförmige Fokuszone, deren Zentrum F in der in Fig. 5 veranschaulichten Weise je nach Phasenversatz der den Ultraschall-Wandlerelementen 1₁ bis 1ₙ zugeführten Signale innerhalb einer etwa rechteckförmigen Zone Z verlagert werden kann. Die Zone z befindet sich in der Ebene, die die Längsachse des Ultraschall-Wandlers 15 sowie den Linienfokus FL enthält.

Die entsprechende zur Ansteuerung der Ultraschall-Wandlerelemente 1₁ bis 1ₙ vorgesehene Generatoreinrichtung 7 ist in Fig. 1 schematisch veranschaulicht und steht mit der Quelle 1 über eine Leitung 8 in Verbindung, die in der Praxis eine an die Anzahl der Ultraschall-Wandlerelemente angepaßte Anzahl von Adern aufweist.

Um die Fokuszone entsprechend den jeweiligen Bedürfnissen innerhalb der Zone Z verlagern zu können, ist an die Generatoreinrichtung 7 ein Joystick 9 angeschlossen.

Die Ultraschall-Ortungseinrichtung, die die Erzeugung von Echtzeit- oder genauer gesagt Quasi-Echtzeit-Ultraschallbildern erlaubt, enthält außer dem Sektor-Scanner 2 ein mit diesem zusammenwirkendes, konventionell aufgebautes Ultraschallgerät 10, das über eine Leitung 11 von der Generatoreinrichtung 7 ein Signal erhält, das der jeweils eingestellten Position des Fokus der Ultraschallwellen entspricht. Auf Grundlage dieses Signals blendet das Ultraschallgerät 10 eine Marke F' in die im Zusammenwirken mit dem Sektor-Scanner 2 erzeugten Ultraschallbilder ein, die die Lage des Zentrums F der Fokuszone in den mittels der Ultraschall-Ortungseinrichtung erzeugten Ultraschallbildern angibt.

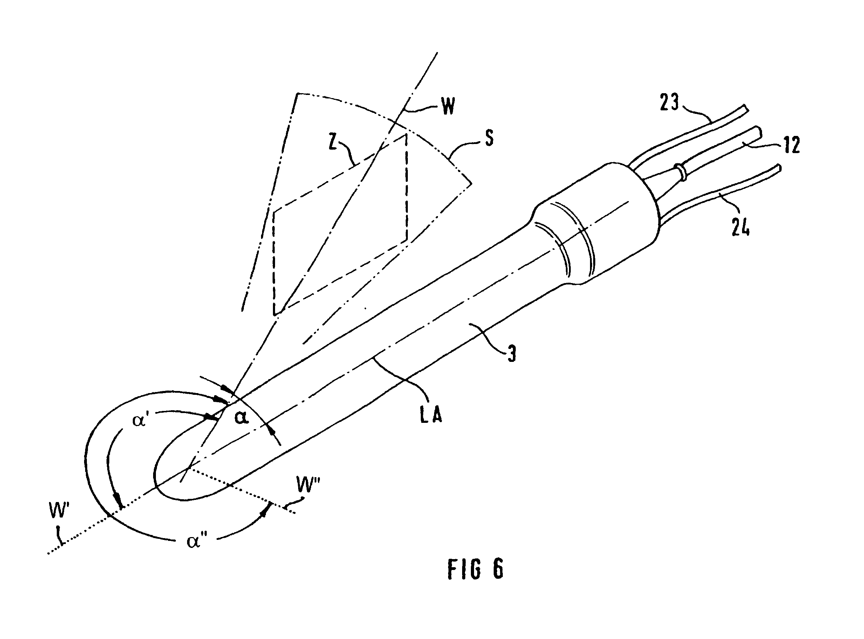

Ein aus dem Gehäuse 3 nach außen geführtes Kabel 12 (siehe Fig. 6) umfaßt einerseits die die Generatoreinrichtung 7 mit den Ultraschall-Wandlerelementen 1₁ bis 1ₙ verbindende Leitung 8 sowie den Sektor-Scanner 2 mit dem Ultraschallgerät 10 verbindenden Leitungen.

Die Quelle 1 und der Sektor-Scanner 2 sind derart in Richtung der Längsachse des Applikators aufeinanderfolgend angeordnet, daß der Sektor-Scanner 2 in Einführungsrichtung des Applikators vor der Quelle 1 am distalen Ende des Applikators angeordnet ist, und zwar so, daß in der aus Fig. 6 ersichtlichen Weise die Mittelebene der mittels des Sektor-Scanners 2 abgetasteten kreissektorförmigen Schicht S in der die Längsachse des Ultraschall-Wandlers 15 sowie den Linienfokus FL enthaltenden Ebene liegt. In dieser Ebene ist wie erwähnt der Fokus der therapeutischen Ultraschallwellen verlagerbar. Die Winkelhalbierende W der mittels des Sektor-Scanners 2 abgetasteten kreissektorförmigen Schicht S, die im Falle des beschriebenen Ausführungsbeispiels mit deren Mittelachse identisch ist, schließt in der aus Fig. 6 ersichtlichen Weise mit dem im Bereich der Quelle 1 befindlichen Abschnitt der Längsachse des Ultraschall-Wandlers 15 und damit dem im Bereich der Quelle 1 befindlichen Abschnitt der Längsachse LA des Applikators einen Winkel α ein, der kleiner als 90° ist.

Da die Winkelhalbierende W der Schnittebene S mit der Längsachse LA des Applikators den Winkel α in einer solchen Weise einschließt, daß die Winkelhalbierende W zu der Quelle 1 hin geneigt ist, wird in dem mittels der Ultraschall-Ortungseinrichtung erzeugten Ultraschallbild stets der gesamte Bereich, d.h. die Zone Z, abgebildet, in dem sich das Zentrum der Fokuszone F befinden kann, und das obwohl der Sektor-Scanner 2 am distalen Ende des Applikators angebracht ist.

Für eine mögliche Position der Fokuszone ist in Fig. 6 das Zentrum der Fokuszone beispielhaft eingetragen und mit F bezeichnet.

Die beschriebene Betriebsweise, bei der der Winkel α kleiner als 90° ist und die Winkelhalbierende W zu der Quelle 1 hin geneigt ist, stellt die Betriebsweise während der Behandlung eines Patienten mittels der von der Quelle 1 ausgehenden Ultraschallwellen dar.

Es ist jedoch eine zweite Betriebsweise möglich, bei der die Winkelhalbierende W der Schnittebene S von dem im Bereich der Quelle 1 befindlichen Abschnitt der Längsachse LA des Applikators weg geneigt und der Winkel zwischen der Winkelhalbierende W und dem im Bereich der Quelle 1 befindlichen Abschnitt der Längsachse LA des Applikators größer als 90° ist. Diese Betriebsweise ist in Fig. 6 dadurch angedeutet, daß für zwei exemplarische Winkel α' und α'' die Winkelhalbierenden W' und W'' punktiert eingetragen sind. Dabei veranschaulichen α' und W' eine Zwischenposition. α'' und W'' entsprechen der einen Endposition. Die andere Endposition ist durch α und W definiert.

Die zweite Betriebsweise dient dazu, während der Einführung des Applikators in einen Patienten mittels der Ultraschall-Ortungseinrichtung den während des Einführvorgangs im Bereich des distalen Endes des Applikators, insbesondere vor dem distalen Ende, befindlichen Körperbereich des Patienten darzustellen, um Informationen zu erhalten, welche es gestatten zu erkennen, ob eine problemlose Einführung möglich ist oder ob beispielsweise die Einführrichtung korrigiert werden muß.

In der Regel wird man bei Beginn der Einführung des Applikators eine Stellung der Winkelhalbierenden der Schnittebene S wählen, in der diese wenigstens annähernd parallel zur Längsachse des Applikators verläuft oder mit dieser zumindest annähernd zusammenfällt. Diese Situation ist in Fig. 6 durch die mit W' bezeichnete Stellung der Winkelhalbierenden und den Winkel α' veranschaulicht. Treten während des Einführvorganges Hindernisse auf oder werden während des Einführvorganges aus anderen Gründen Informationen über den das distale Ende des Applikators umgebenden Körperbereich des Patienten benötigt, kann die Winkelhalbierende zwischen den Winkeln α und α'' beliebig verstellt werden, um geeignete Ultraschallbilder gewinnen zu können, wobei vorzugsweise α''=360°- 2α gilt.

Wie aus den Fig. 1 bis 3 ersichtlich ist, weist der Sektor-Scanner 2 einen in seiner Gestalt der Innenkontur des Gehäuses 3 angepaßten Grundkörper 13 auf, der mit einer schlitzförmigen Ausnehmung versehen ist, in der ein Rotor 14 aufgenommen ist, an dem ein Ultraschall-Transducer 15 angebracht ist.

Der Rotor 14 ist auf einer in eine Öffnung des Grundkörpers 13 eingepreßten Achse 16 mittel eines nicht dargestellten Gleitlagers drehbar gelagert.

Der Rotor 14 ist ohne Zwischenschaltung eines Getriebes mittels eines druckmittelbetriebenen Motors angetrieben, und zwar unter Verwendung des in dem Ultraschall-Applikator enthaltenen Mineralöls als Druckmittel.

Dieser Motor ist nach Art einer Turbine aufgebaut und weist als wesentliches Element ein fest an dem Rotor 14 angebrachtes, mit einer Beschaufelung 17 versehenes Turbinenrad 18 auf, das in einer der die Achse 16 aufnehmenden Öffnung gegenüberliegenden Öffnung 19 des Grundkörpers 13 aufgenommen ist, die nur wenig größer als der Außendurchmesser des Turbinenrades 18 ist.

In dem Grundkörper 13 erstreckt sich ein Kanal 20, der so angeordnet ist, daß er durch die Öffnung 19 in zwei Abschnitte nämlich einen Zulaufabschnitt 20a und einen Rücklaufabschnitt 20b unterteilt ist, wobei der Zulaufabschnitt so angeordnet ist, daß als Druckmittel durch den Kanal 20 geleitetes Mineralöl unter einer geeigneten Strömungsrichtung, im Falle des beschriebenen Ausführungsbeispiels tangential, auf die Beschaufelung 17 des Turbinenrades 18 trifft und dieses samt des Rotors 14 und des Ultraschall-Transducers 15 in Rotation versetzt.

Dem Zulaufabschnitt 20a ist das Mineralöl über eine sich durch den Tragkörper 5 der Quelle 1 erstreckenden Kanal 21 zugeführt. Auf diese Weise dient das Mineralöl zugleich der Kühlung der Quelle 1. Von dem Rücklaufabschnitt 20b gelangt das Mineralöl über eine in dem Grundkörper 13 vorgesehene Nut 22 zu einer Leitung 23 siehe Fig. 6. Der Kanal 21 wird von einer ebenfalls in Fig. 6 dargestellten Leitung 24 gespeist. Die Leitungen 23 und 24 bilden einen Kreislauf, in den eine Pumpe 27 mit regelbarer Förderleistung (Druck bzw. Durchflußmenge) eingefügt ist. Falls erforderlich, kann der Kreislauf in der in Fig. 1 dargestellten Weise ein Kühlaggregat 36 für das Mineralöl enthalten.

Der Ultraschall-Transducer 15 wirkt wie bereits erläutert mit dem einen Monitor 25 enthaltenden Ultraschallgerät 10 zusammen. Der hierzu erforderliche Austausch von elektrischen Signalen erfolgt über Schleifringe 28 und 29 sowie Schleifkontakte 30 und 31, wobei über den Schleifring 28 und den Schleifkontakt 30 die eigentlichen Signale laufen und der Schleifring 29 mit dem Schleifkontakt 31 die Masseverbindung herstellt. Die entsprechenden Leitungen verlaufen wie erwähnt in dem Kabel 12.

Für die ordnungsgemäße Erzeugung von Ultraschall-Bildern benötigt die Ultraschall-Bilderzeugungseinheit 17 auch ein Signal bezüglich der Winkelstellung des Rotors 14. Dieses wird dadurch erzeugt, daß der Schleifring 29 in gleichen Winkelabständen voneinander angeordnete axial gerichtete Fortsätze 29₁ bis 29ₙ n= 1 bis 400 aufweist, die mittels eines weiteren Schleifkontaktes 32 abgetastet werden, der über einen Widerstand 33 mit einer positiven Spannung U+ und eine in dem Kabel 12 verlaufende Leitung mit der Ultraschall-Bilderzeugungseinheit 17 verbunden ist. Um ein Referenzsignal für eine bestimmte Winkelstellung des Rotors 14, beispielsweise diejenige, in der zwischen der Winkelhalbierenden und der Längsachse des Applikators ein Winkel von 90° vorliegt, zu erzeugen, ist einer der Fortsätze, z.B. wie dargestellt der Fortsatz 29₁, länger als die anderen ausgeführt. Er wird mittels eines Schleifkontaktes 37 abgetastet. Auch dieser ist über einen Widerstand 38 mit der positiven Spannung U+ und eine in dem Kabel 12 verlaufende Leitung mit der Ultraschall-Bilderzeugungseinheit 17 verbunden.

Das an dem Schleifkontakt 32 zur Verfügung stehende Rechtecksignal ist nicht nur der Ultraschall-Bilderzeugungseinheit 17, sondern außerdem auch einer unter Verwendung von fuzzy logic aufgebauten Drehzahlregeleinrichtung 34 zugeführt, die die Förderleistung der Pumpe 27 im Sinne einer definierten, konstanten Winkelgeschwindigkeit des Rotors 14, also einer konstanten Frequenz des Rechtecksignals, regelt.

Um die Stellung der Winkelhalbierenden der Schnittebene S einstellen zu können ist eine Verstelleinheit 39 an Ultraschallgerät 10 angeschlossen, die einen Stellknopf 40 aufweist mittels dessen anhand einer Skala die Winkelhalbierende in die der ersten Betriebsweise entsprechend Stellung (W, α) gebracht werden kann. Die entsprechende Stellung des Stellknopfes 40 ist auf der Skala mit T bezeichnet. Derjenigen Stellung (W', α') der Winkelhalbierenden, in der diese parallel zur Längsachse LA des Applikators verläuft, entspricht die auf der Skala die mit LA bezeichnete Stellung des Stellknopfes 40. Die der der in der ersten Betriebsweise vorliegenden Stellung der Winkelhalbierenden entgegengesetzten Stellung (W'', α'') der Winkelhalbierenden entspricht die auf der Skala die mit α'' bezeichnete Stellung des Stellknopfes 40.

Das Ultraschallgerät 10 stellt anhand der von den Schleifkontakten 32 und 37 stammenden Signale die Stellung der Winkelhalbierenden der Schnittebene S entsprechend der Einstellung des Stellknopfes 40 der Stelleinheit 39 ein.

Zur Durchführung einer Behandlung wird so vorgegangen, daß der Applikator zunächst in die entsprechende Körperöffnung, z. B. das Rektum eines männlichen Patienten, eingeführt wird. Dies geschieht unter Ultraschallkontrolle mit der zunächst in der zweiten Betriebsweise arbeitenden Ultraschall-Ortungseinrichtung. Dabei ist der Stellknopf 40 der Verstelleinheit 39 zunächst auf die Stellung LA gestellt. Sofern erforderlich können während des Einführungsvorgangs mittels des Stellknopfes 40 auch andere Positionen der Winkelhalbierenden der Schnittebene S gewählt werden. Ist der Einführungsvorgang abgeschlossen wird die Ultraschall-Ortungseinrichtung in die erste Betriebsweise gebracht, indem der Stellknopf 40 auf die Stellung T gebracht wird. Dann wird der Applikator mit Hilfe der Ultraschall-Ortungseinrichtung anhand des auf deren Monitor 25 dargestellten Ultraschallbildes derart ausgerichtet, daß sich der zu behandelnde Körperbereich, z. B. eine gutartig vergrößerte Prostata, sich etwa im Zentrum des Ultraschallbildes befindet. Der Applikator wird nun in nicht näher dargestellter Weise mittels eines Stativs oder dergleichen in dieser Lage fixiert.

Anschließend wird mittels des Joysticks 9 der Fokus der Ultraschallwellen auf einen zu behandelnden Bereich der Prostata ausgerichtet.

Wird nun der Schalter 35 (siehe Fig. 1), bei dem es sich beispielsweise um einen Fußschalter handeln kann, betätigt, so sendet die Quelle 1 einen Ultraschallimpuls aus, dessen Amplitude und Zeitdauer so gewählt sind, daß das im Fokus der Ultraschallwellen befindliche Prostatagewebe auf so hohe Temperaturen erwärmt wird, daß dies eine Denaturierung des Zelleiweißes und damit eine Nekrotisierung des betroffenen Prostatagewebes nach sich zieht.

Der beschriebene Vorgang kann nun, evtl. automatisch, unter jeweils geringfügiger Verschiebung des Fokus der Ultraschallwellen so lange wiederholt werden, bis die gesamte Prostata oder ein bestimmter Bereich der Prostata behandelt ist.

Der im Zusammenhang mit dem Ausführungsbeispiel gemäß den Fig. 1 bis 6 beschriebene Aufbau des druckmittelbetriebenen Motors als Turbine ist nur beispielhaft zu verstehen; der druckmittelbetriebene Motor kann auch andersartig, z.B. als Drehkolbenmotor, aufgebaut sein.

Anstelle eines druckmittelbetriebenen Motors kann auch ein anderer Motor, der den getriebelosen Antrieb des Rotors ermöglicht, vorgesehen sein, z.B. ein piezoelektrischer Außenläufermotor oder ein elektromagnetischer Außenläufermotor.

Bei dem vorstehend beschriebenen Ausführungsbeispiel ist als Quelle therapeutischer akustischer Wellen eine Ultraschallguelle vorgesehen. Diese kann als therapeutische akustische Wellen Ultraschall in Form von Ultraschallimpulsen oder in Form von Dauerschall abgeben. Anstelle einer Ultraschallquelle kann aber auch eine andere Quelle therapeutischer akustischer Wellen vorgesehen sein, z.B. eine Stoßwellenquelle.

Im Falle des beschriebenen Ausführungsbeispiels ist als Sektor-Scanner ein mechanischer Sektor-Scanner vorgesehen. Es besteht aber auch die Möglichkeit, statt dessen einen elektronischen Sektor-Scanner vorzusehen, der ein Phased Array enthält, wobei es in diesem Falle unter Umständen erforderlich sein kann, das Phased Array schwenkbar anzuordnen, sofern der elektronisch realisierbare Schwenkbereich der Winkelhalbierenden bzw. Mittelachse der Schnittebene S nicht ausreicht.

## Patentansprüche

1. Akustisches Therapiegerät, aufweisend eine Quelle (1) therapeutischer akustischer Wellen und eine Ultraschall-Ortungseinrichtung mit einem Ultraschall-Transducer (15), wobei die Quelle (1) und der Ultraschall-Transducer (15) derart zu einem länglichen Applikator mit einer Längsachse zusammengefaßt sind, daß die Quelle (1) und der Ultraschall-Transducer (15) in Richtung der Längsachse aufeinanderfolgend angeordnet sind, wobei mittels der Ultraschall-Ortungseinrichtung Ultraschall-Bilder bezüglich einer kreissektorförmigen, die Längsachse des Applikators enthaicenden Schnittebene (S) erzeugbar sind, deren Mittelachse (W) die Winkelhalbierende der Schnittebene (S) ist wobei in einer ersten Betriebsposition die Mittelachse (W) der Schnittebene (S) mit dem im Bereich der Quelle (1) befindlichen Abschnit der Längsachse des Applikators einen Winkel (α) einschließt, der kleiner als 90° ist und somit die Mittelachse (W) zu der Quelle, (1) hin geneigt ist, **dadurch gekenn-zeichnet, daß** der Ultraschall-Transducer (15) in Einführrichtung vor der Quelle (1) angeordnet ist und daß die Mittelachse (W) derart verstellbar ist, daß sie in einer weiteren Betriebsposition mit dem im Bereich der Quelle (1) befindlichen Abschnitt der Längsachse des Applikators einen zweiten Winkel (α', α'') einschließt, der größer als 90° ist.

2. Akustisches Therapiegerät nach Anspruch 1, dessen Applikator solche Abmessungen aufweist, daß er auf natürlichem Wege in das Körperinnere eines menschlichen Patienten einführbar ist.

3. Akustisches Therapiegerät nach Anspruch 1 oder 2, bei dem als Quelle (1) therapeutischer akustischer Wellen eine Ultraschallquelle vorgesehen ist.

4. Akustisches Therapiegerät nach Anspruch 3, dessen Ultraschallquelle von langgestreckter Gestalt ist.

5. Akustisches Therapiegerät nach Anspruch 4, dessen Ultraschallquelle als linear array ausgeführt ist.

## Claims

1. Acoustic therapy apparatus comprising a source (1) of therapeutic acoustic waves and an ultrasound locating system with an ultrasound transducer (15), said source (1) and the ultrasound transducer (15) being combined to form an oblong applicator with a longitudinal axis, the source (1) and the ultrasound transducer (15) being successively arranged in the direction of the longitudinal axis, said ultrasound locating system generating ultrasound images with respect to a circular planar section (S) that contains the longitudinal axis of the applicator and whose centre axis (W) is the angle bisector of the planar section (S), whereby the centre axis (W) of the planar section (S) describes, in a first operating position, an angle (α) of less than 90° with the section of the longitudinal axis of the applicator located in the source (1), the centre axis (W) thus being inclined towards the source (1), **characterised in that** the ultrasound transducer (15) is disposed in the direction of introduction preceding the source (1) and that the centre axis (W) can be adjusted such that in a further operating position it describes a second angle (α', α") greater than 90° with the section of the longitudinal axis of the applicator located in the source (1).

2. Acoustic therapy apparatus according to Claim 1, the applicator of which comprises dimensions enabling said applicator to be introduced into the body interior of a human patient along natural paths.

3. Acoustic therapy apparatus according to Claim 1, in which the source (1) of therapeutic acoustic waves comprises an ultrasound source.

4. Acoustic therapy apparatus according to Claim 3, the ultrasound source of which has an elongated shape.

5. Acoustic therapy apparatus according to Claim 4, the ultrasound source of which comprises a linear array.

## Revendications

1. Appareil thérapeutique acoustique qui présente une source (1) d'ondes acoustiques thérapeutiques et une installation de localisation à ultrasons comportant un transducteur d'ultrasons (15), dans lequel la source (1) et le transducteur d'ultrasons (15) sont regroupés en un applicateur oblong comportant un axe longitudinal de telle manière que la source (1) et le transducteur d'ultrasons (15) sont disposés l'un derrière l'autre en direction de l'axe longitudinal, dans lequel on peut produire à l'aide de l'installation de localisation à ultrasons des images ultrasonores par rapport à un plan de coupe (S) en secteur de cercle qui passe par l'axe longitudinal de l'applicateur et dont l'axe médian (W) est la bissectrice du plan de coupe (S), dans lequel dans un premier mode de fonctionnement l' axe médian (W) du plan de coupe (S) fait avec le segment - qui se trouve dans la zone de la source (1) - de l'axe longitudinal de l'applicateur un angle (α) inférieur à 90° et l'axe médian (W) est par conséquent incliné vers la source (1),
**caractérisé en ce que** le transducteur d'ultrasons (15) est disposé en direction d'introduction devant la source (1) et l'axe médian (W) peut être déplacé de telle sorte que dans une autre position de fonctionnement il fait avec le segment - qui se trouve dans la zone de la source (1) - de l'axe longitudinal de l'applicateur un deuxième angle (α', α'') supérieur à 90°.

2. Appareil thérapeutique acoustique selon la revendication 1, dont l'applicateur présente des dimensions telles qu'il peut être introduit par des voies naturelles à l'intérieur du corps d'un patient humain.

3. Appareil thérapeutique acoustique selon la revendication 1 ou 2, pour lequel on prévoit une source ultrasonore en tant que source (1) d'ondes acoustiques thérapeutiques.

4. Appareil thérapeutique acoustique selon la revendication 3, dont la source ultrasonore a une structure oblongue.

5. Appareil thérapeutique acoustique selon la revendication 4, dont la source ultrasonore est réalisée en réseau linéaire.
